# EUROPÄISCHE PATENTSCHRIFT

(11) **EP 3 880 842 B1**
(45) Veröffentlichungstag und Bekanntmachung des Hinweises auf die Patenterteilung: **26.04.2023**
(21) Anmeldenummer: 19804666.6
(22) Anmeldetag: 08.11.2019
(51) Int. Cl.: G01N 21/27, G01N 21/64, C12Q 1/686

(54) **VERFAHREN UND VORRICHTUNG ZUR ANALYSE VON BIOLOGISCHEM MATERIAL**
METHOD AND DEVICE FOR ANALYZING BIOLOGICAL MATERIAL
PROCÉDÉ ET DISPOSITIF D'ANALYSE D'UN MATÉRIAU BIOLOGIQUE

(30) Priorität: 15.11.2018 DE 102018219483
(43) Veröffentlichungstag der Anmeldung: 22.09.2021
(73) Patentinhaber: Robert Bosch GmbH, 70442 Stuttgart (DE)
(72) Erfinder: FRANK, Tino, 6003 Luzern (CH)
(86) Internationale Anmeldenummer: PCT/EP2019/080713
(87) Internationale Veröffentlichungsnummer: WO 2020/099269

(56) Entgegenhaltungen:
- EP-A1- 2 166 470
- EP-A2- 1 770 172
- US-A1- 2006 009 916
- US-A1- 2007 073 489

## Beschreibung

### Stand der Technik

Die Erfindung geht von einer Vorrichtung oder einem Verfahren nach Gattung der unabhängigen Ansprüche aus. Gegenstand der vorliegenden Erfindung ist auch ein Computerprogramm.

Eine Herausforderung bei mikrofluidischen System kann beispielsweise in einer Vermeidung oder Kontrolle von Luftblasen bestehen. Insbesondere bei Anwendung von Temperaturen in der Nähe des Siedepunktes können solche Luftblasen vermehrt entstehen. Beispielsweise kann eine Polymerase-Kettenreaktion Prozessschritte erfordern, bei denen Temperaturen in diesem Bereich eingesetzt werden. Da hierbei eine Analyse oder Auswertung üblicherweise über optische Signale erfolgt, können solche Luftblasen eine Störquelle darstellen, welche insbesondere ein Rauschen gemessener Intensitätswerte verursachen kann.

Aus den Dokumenten EP 2 166 470 A1 und EP 1 770 172 A2 sind Verfahren zur Analyse von biologischem Material, insbesondere von PCR-Daten, bekannt.

### Offenbarung der Erfindung

Vor diesem Hintergrund werden mit dem hier vorgestellten Ansatz ein Verfahren, weiterhin eine Vorrichtung, welche dieses Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Gemäß Ausführungsformen kann insbesondere eine Amplifikationsentscheidung für eine rauschintensive, mikrofluidische bzw. optofluidische Analyse von biologischem Material auf zuverlässige Weise ermöglicht werden. Insbesondere kann hierbei eine Auswertung von optofluidischen Daten trotz durch Gasblasen verursachten Rauschens durchgeführt werden. Eine solche Analyse kann beispielsweise eine sogenannte quantitative Echtzeit-PCR (qPCR; PCR = polymerase chain reaction bzw. Polymerase-Kettenreaktion) sein. Dabei kann insbesondere ein Vorhandensein von kleinsten Mengen eines spezifischen DNA-Abschnittes (DNA = Desoxyribonukleinsäure) sowohl quantitativ als auch qualitativ nachgewiesen werden. Somit kann gemäß Ausführungsformen insbesondere ein Prozess zur Amplifikationsentscheidung für rauschintensive, mikrofluidische qPCR-Analysen bereitgestellt werden. Bei der Amplifikationsentscheidung kann entschieden werden, ob Messdaten auf eine Amplifikation von durch die Analyse nachzuweisenden Molekülen hindeuten oder nicht.

Mikrofluidische Systeme können eine Analyse von kleinen Probenmengen mit einer hohen Sensitivität ermöglichen. Automation, Miniaturisierung und Parallelisierung können bei mikrofluidischen Systemen zudem eine Reduktion von händischen Schritten, sowie eine Verminderung von dadurch verursachten Fehlern ermöglichen. Mikrofluidische Systeme, welche fluidische und analytische Vorgänge über ein optisches System ausführen können, werden insbesondere der Optofluidik zugeordnet. Optofluidische Prozesse können dabei Eigenschaften von Licht nutzen, um Resultate zu generieren. Bei der Analyse aufgrund von Gasblasen bzw. Luftblasen entstehendes Rauschen kann insbesondere auch ohne eine aufwendige und nicht zielführende Verbesserung von Signalhub oder Sondendesign berücksichtigt und herausgerechnet werden. Somit kann beispielsweise verhindert werden, dass ein Auswerten von solchen verrauschten Messdaten vereinfacht werden kann und eine durch solche Blasen verursachte Verringerung von Spezifität sowie Sensivität der Analyse verhindert werden kann. Um diese wichtigen Größen der Analyse und somit des zugrundeliegenden Diagnosesystems trotz Blaseninterferenz aufrechtzuerhalten, können insbesondere vorteilhafte Signalverarbeitungsverfahren eingesetzt werden.

Vorteilhafterweise kann gemäß Ausführungsformen insbesondere ein unvoreingenommener (unbiased) Entscheidungsprozess hinsichtlich eines Ergebnisses der Analyse erreicht werden. So kann beispielsweise auf eine Ausgleichsrechnung (engl. curve fitting) an zwei zu erwartende Modelle - in der qPCR sind dies eine lineare Funktion oder eine sigmoidale Funktion - verzichtet werden. Anders als bei einer Ausgleichsrechnung, bei welcher der kleinere Fehler (engl. Goodness of fit) als das vorliegende Modell genommen würde, d. h. den Entscheid repräsentieren würde, ob das Verhalten der Daten einer linearen Funktion oder einer sigmoidalen Funktion entspricht, kann gemäß Ausführungsformen insbesondere auch bei hohem Rauschen, bei dem Residuen (Fehler der Ausgleichsrechnung) zu nahe beieinanderlägen, ein statistisch relevanter Unterschied erhalten werden. Zudem können erforderliche Testdaten verringert werden, um Akzeptanzkriterien zu definieren und zu validieren. Durch Messen des Rauschens und Erzeugen von rauschanalogen, generischen Modelldaten, kann auf Testdaten auch verzichtet werden. Nach einem Modellentscheid kann ein Fitting durchgeführt werden, was eine einfache Anpassung des Ausgleichsrechnungsprinzips ermöglicht. Es kann trotz Blasenbildung während einer Analyse, insbesondere eine qPCR, in einem mikrofluidischen System bzw. in einer mikrofluidischen Vorrichtung ein zuverlässiger Amplifikationsentscheid mit einem minimalen Einsatz von Kontrollreaktionen gefällt werden. Die Analyse kann vollautomatisiert ablaufen und kommt ohne Nutzereingabe aus. Es brauchen keine zusätzlichen, analysespezifischen Parameter, außer den Messdaten selbst, zur Auswertung eingegeben werden. Durch eine Universalität der vorgestellten Methode eignet sich diese besonders in qPCR-Systemen am Einsatzort bzw. Point-of-care. Benutzungsfehler durch falsche Parameterwahl können minimiert bzw. verhindert werden.

Es wird ein Verfahren zur Analyse von biologischem Material vorgestellt, wobei das Verfahren folgende Schritte aufweist:
Einlesen eines Messsignals, eines ersten Referenzsignals und eines zweiten Referenzsignals, wobei das Messsignal erfasste optofluidische Daten des biologischen Materials repräsentiert, wobei das erste Referenzsignal erste optofluidische Modelldaten repräsentiert, die einer ersten Eigenschaft des biologischen Materials entsprechen, wobei das zweite Referenzsignal zweite optofluidische Modelldaten repräsentiert, die einer zweiten Eigenschaft des biologischen Materials entsprechen;
Ermitteln eines Rauschens des Messsignals, um Rauschdaten zu erzeugen;
Anwenden der Rauschdaten auf das erste Referenzsignal und auf das zweite Referenzsignal, um ein angepasstes erstes Referenzsignal und ein angepasstes zweites Referenzsignal zu generieren;
Transformieren des Messsignals, des angepassten ersten Referenzsignals und des angepassten zweiten Referenzsignals in eine Häufigkeitsverteilungsform, um eine Messsignalverteilung, eine erste Referenzverteilung und eine zweite Referenzverteilung zu erzeugen; und
Durchführen einer Clusteranalyse unter Verwendung der Messsignalverteilung, der ersten Referenzverteilung und der zweiten Referenzverteilung, um abhängig von einem Resultat der Clusteranalyse festzustellen, ob das biologische Material die erste Eigenschaft oder die zweite Eigenschaft aufweist.

Bei dem biologischem Material kann es sich um eine Zielmoleküle, beispielsweise DNA-Abschnitte, umfassende Flüssigkeit handeln. Das Verfahren kann unter Verwendung eines mikrofluidischen Systems oder einer mikrofluidischen Vorrichtung ausgeführt werden. Das Messsignal kann ein unter Verwendung eines optischen Sensors erfasstes Signal repräsentieren. Eine Charakteristik des Messsignals kann von einer Eigenschaft des biologischen Materials abhängig sein. Eine Eigenschaft des biologischen Materials kann beispielsweise von einer Art und/oder Konzentration von Zielmolekülen in dem biologischen Material bestimmt sein. Ein Referenzsignal kann ein gespeichertes Signal sein. Die optofluidischen Modelldaten eines Referenzsignals können beispielsweise vorab in einer Referenzmessung erfasst worden sein oder vorab simuliert oder berechnet worden sein. Das erste Referenzsignal kann einer ersten Art oder Menge eines Zielmoleküls entsprechen. Das zweite Referenzsignal kann einer zweiten Art oder Menge eines Zielmoleküls entsprechen. Auch kann das erste Referenzsignal einem Vorhandensein eines Zielmoleküls entsprechen und kann das zweite Referenzsignal einem Nichtvorhandensein des Zielmoleküls entsprechen, wobei ein Vorhandensein beispielsweise durch eine über einem Schwellenwert liegende Konzentration des Zielmoleküls in dem biologischen Material definiert sein kann. Im Schritt des Ermittelns können die Rauschdaten beispielsweise durch eine geeignete Filterung des Messsignals erzeugt werden. Im Schritt des Anwendens können die Rauschdaten und die Referenzsignale kombiniert werden. Beispielsweise können die Referenzsignale mit den Rauschdaten überlagert werden. Die angepassten Referenzsignale können eine dem Messsignal entsprechende Rauschcharakteristik aufweisen. Im Schritt des Durchführens kann ein Analysesignal bestimmt werden, das das Resultat der Clusteranalyse abbilden kann und somit die unter Verwendung des Messsignals und den Referenzsignalen bestimmte Eigenschaft des biologischen Materials anzeigen kann.

Eine Ausführung des Verfahrens kann einen Entscheidungsprozess basierend auf einer Clusteranalyse bzw. einem Clusterverfahren ermöglichen. Dabei kann ein gemessenes Signal mit zwei generischen, resultatetypischen Referenzsignalen verglichen werden. Um die Clusteranalyse zu vereinfachen, kann das Rauschen der Messdaten gemessen werden und können Referenzdaten mit demselben Rauschen erzeugt werden. Das Transformieren der Signale in eine Verteilungsform ermöglicht eine robustere Entscheidung hinsichtlich eines Ergebnisses der Analyse.

Gemäß einer Ausführungsform weist das Verfahren einen Schritt des Erfassens der optofluidischen Daten des biologischen Materials auf, um das Messsignal bereitzustellen. Dabei kann der Schritt des Erfassens mittels mikrofluidischer Einrichtungen ausgeführt werden. Eine solche Ausführungsform bietet den Vorteil, dass auch kleine Mengen an biologischem Material mit hoher Sensitivität auf automatische und miniaturisierte Weise analysiert werden können.

Auch kann im Schritt des Einlesens ein Messsignal eingelesen werden, das mittels einer quantitativen und zusätzlich oder alternativ qualitativen Polymerase-Kettenreaktion erfasste optofluidische Daten des biologischen Materials repräsentiert. Hierbei werden im Schritt des Einlesens ein erstes Referenzsignal, das einen sigmoidalen Verlauf aufweist, und ein zweites Referenzsignal, das einen linearen Verlauf aufweist, eingelesen. Dabei kann die erste Eigenschaft des biologischen Materials zu einer Amplifikation zumindest eines Zielmoleküls des biologischen Materials durch die Analyse führen. Hierbei kann die zweite Eigenschaft des biologischen Materials zu einem Ausbleiben einer Amplifikation des zumindest einen Zielmoleküls durch die Analyse führen. Dabei werden im Schritt des Transformierens das angepasste erste Referenzsignal und das angepasste zweite Referenzsignal derart transformiert, dass die erste Referenzverteilung eine bimodale Verteilung ist und die zweite Referenzverteilung eine unimodale Verteilung ist. Eine solche Ausführungsform bietet den Vorteil, dass eine Auswertung bzw. Amplifikationsentscheidung für eine Polymerase-Kettenreaktion unaufwendig, sicher und genau durchgeführt werden kann.

Ferner kann im Schritt des Durchführens die Clusteranalyse mittels eines k-Means-Algorithmus mit einem vordefinierten Distanzmaß durchgeführt werden. Hierbei kann die erste Referenzverteilung ein erstes Cluster repräsentieren. Die zweite Referenzverteilung kann ein zweites Cluster repräsentieren. Dabei kann das Resultat der Clusteranalyse angeben, ob unter Berücksichtigung des Distanzmaßes die Messsignalverteilung in das erste Cluster oder in das zweite Cluster fällt. Anstelle des k-Means-Algorithmus kann auch ein anderer Algorithmus aus dem Gebiet der Clusteranalyse oder einem geeigneten anderen Gebiet verwendet werden. Eine solche Ausführungsform bietet den Vorteil, dass eine zuverlässige Amplifikationsentscheidung für die Analyse an dem biologischen Material auf einfache Art und Weise getroffen werden kann. Zudem kann im Schritt des Ermittelns das Rauschen des Messsignals über einen Verlauf der Analyse hinweg und zusätzlich oder alternativ mittels eines Schiebefensterprozesses und zusätzlich oder alternativ unter Verwendung eines Rauschmaßes ermittelt werden, um als die Rauschdaten einen funktionalen Zusammenhang zwischen dem Rauschen und dem Verlauf der Analyse zu erzeugen. Das Rauschmaß kann eine lokale Standardabweichung, ein lokales Signal-zu-Rausch-Verhältnis oder dergleichen sein. Der Verlauf der Analyse kann einen Ablauf der Zeit und zusätzlich oder alternativ eine Zyklenzahl der Analyse repräsentieren. Eine solche Ausführungsform bietet den Vorteil, dass eine Entscheidung, ob das Messsignal eher dem ersten Referenzsignal oder dem zweiten Referenzsignal entspricht, noch robuster und zuverlässiger getroffen werden kann.

Im Schritt des Anwendens können von den Rauschdaten abhängige Zufallszahlen oder Pseudozufallszahlen zu den Modelldaten der Referenzsignale addiert werden. Eine solche Ausführungsform bietet den Vorteil, dass die angepassten Referenzsignale auf einfache Weise erhalten werden können, um das in dem Messsignal vorhandene Rauschen genau abzubilden.

Das Verfahren kann ferner einen Schritt des Normierens des eingelesenen Messsignals durch Projizieren von Absolutwerten auf ein vordefiniertes Werteintervall aufweisen. Hierbei kann im Schritt des Ermittelns das Rauschen des normierten Messsignals ermittelt werden. Eine solche Ausführungsform bietet den Vorteil, dass die Clusteranalyse erleichtert werden kann bzw. ein exakteres Resultat der Clusteranalyse ermöglicht werden kann.

Dieses Verfahren kann beispielsweise in Software oder Hardware oder in einer Mischform aus Software und Hardware beispielsweise in einem Steuergerät implementiert sein.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrundeliegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EEPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Die Vorrichtung kann als eine mikrofluidische Vorrichtung ausgeführt sein, insbesondere als ein sogenanntes Chiplabor bzw. LoC (Lab-on-Chip). Alternativ kann die Vorrichtung als ein Steuergerät innerhalb oder außerhalb einer mikrofluidischen Vorrichtung ausgeführt sein. In einer vorteilhaften Ausgestaltung erfolgt durch die Vorrichtung eine Steuerung einer mikrofluidischen Vorrichtung. Auch kann die Vorrichtung ausgebildet sein, um eine Auswertung von mittels mikrofluidischer Einrichtungen erfassten Messdaten zu führen. Hierzu kann die Vorrichtung beispielsweise auf Sensorsignale wie das Messsignal zugreifen. Die Vorrichtung kann ausgebildet sein, um ein Ausgangssignal bereitzustellen, das ein Ergebnis oder Zwischenergebnis der Analyse repräsentiert. Bei dem Ergebnis oder Zwischenergebnis kann es sich um eine Aussage darüber handeln, ob das biologische Material die erste Eigenschaft oder die zweite Eigenschaft aufweist.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:
Fig. 1 eine schematische Darstellung einer Vorrichtung gemäß einem Ausführungsbeispiel;
Fig. 2A bis Fig. 2C schematische Signaldiagramme gemäß einem Ausführungsbeispiel;
Fig. 3A bis Fig. 3C schematische Signaldiagramme gemäß einem Ausführungsbeispiel;
Fig. 4A bis Fig. 4D schematische Signaldiagramme gemäß einem Ausführungsbeispiel;
Fig. 5A und Fig. 5B schematische Signaldiagramme gemäß einem Ausführungsbeispiel;
Fig. 6A und Fig. 6B schematische Signaldiagramme gemäß einem Ausführungsbeispiel;
Fig. 7A bis Fig. 7C schematische Signaldiagramme gemäß einem Ausführungsbeispiel;
Fig. 8 ein Ablaufdiagramm eines Auswerteprozesses gemäß einem Ausführungsbeispiel; und
Fig. 9 ein Ablaufdiagramm eines Verfahrens gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

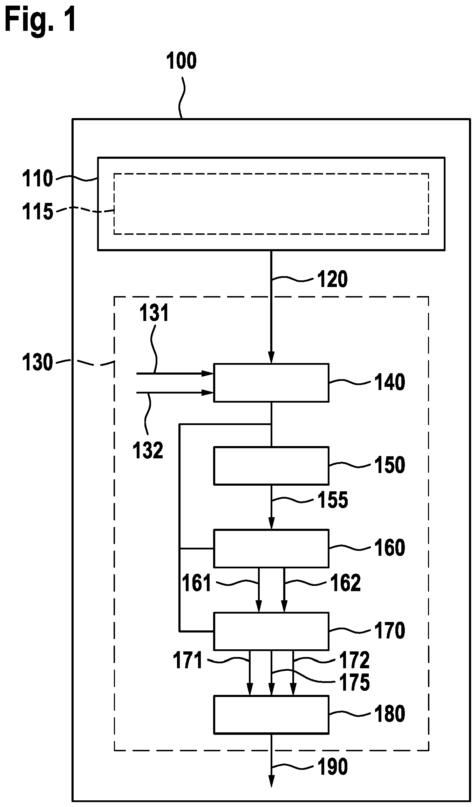

Fig. 1 zeigt eine schematische Darstellung einer Vorrichtung 100 gemäß einem Ausführungsbeispiel. Die Vorrichtung 100 ist ausgebildet, um eine Analyse von biologischem Material auszuführen. Die Vorrichtung 100 kann auch als eine Analysevorrichtung 100 bezeichnet werden. Das biologische Material weist beispielsweise Erbmaterial auf. Die Vorrichtung 100 ist insbesondere als ein Chiplabor ausgeführt. Hierbei ist die Vorrichtung 100 genauer gesagt ausgebildet, um eine quantitative Echtzeit-PCR (qPCR; PCR = polymerase chain reaction bzw. Polymerase-Kettenreaktion) auszuführen. Die qPCR wird hierbei in einer Mehrzahl von Zyklen ausgeführt. Dabei wird beispielsweise ein Vorhandensein von kleinsten Mengen eines spezifischen DNA-Abschnittes (DNA = Desoxyribonukleinsäure) quantitativ und/oder qualitativ nachgewiesen.

Die Vorrichtung 100 weist zumindest eine mikrofluidische Einrichtung 110 auf. Das biologische Material ist beispielsweise in einer Kartusche 115 in die Vorrichtung 100 bzw. mikrofluidische Einrichtung 110 einbringbar. Die Kartusche 115 ist in der Vorrichtung 100 bzw. der mikrofluidischen Einrichtung 110 aufnehmbar. Die zumindest eine mikrofluidische Einrichtung 110 ist ausgebildet, um optofluidische Daten des biologischen Materials zu erfassen. Dazu ist die mikrofluidische Einrichtung 110 beispielsweise ausgebildet, um von dem biologischen Material reflektiertes und/oder ausgesendetes Licht zu erfassen. Ferner ist die zumindest eine mikrofluidische Einrichtung 110 ausgebildet, um ein Messsignal 120 bereitzustellen, beispielsweise unter Verwendung des von dem biologischen Material ausgehenden Lichts. Das Messsignal 120 repräsentiert gemäß einem Ausführungsbeispiel erfasste optofluidische Daten des biologischen Materials. Zum Anregen des biologischen Materials ist die mikrofluidische Einrichtung 110 gemäß einem Ausführungsbeispiel ausgebildet, um das biologischen Material mit elektromagnetischer Strahlung, beispielsweise Licht, zu bestrahlen.

Die Vorrichtung 100 weist zudem ein Steuergerät 130 auf, das auch als Steuereinheit 130 oder Steuervorrichtung 130 zum Analysieren des biologischen Materials bezeichnet wird. Das Steuergerät 130 ist mit der zumindest einen mikrofluidischen Einrichtung 110 signalübertragungsfähig verbunden. Das Steuergerät 130 weist gemäß dem hier dargestellten Ausführungsbeispiel eine Einleseeinrichtung 140, eine Ermittlungseinrichtung 150, eine Anwendungseinrichtung 160, eine Transformationseinrichtung 170 und eine Durchführungseinrichtung 180 auf.

Die Einleseeinrichtung 140 ist ausgebildet, um das Messsignal 120 einzulesen. Ferner ist die Einleseeinrichtung 140 ausgebildet, um ein erstes Referenzsignal 131 und ein zweites Referenzsignal 132 einzulesen. Dabei repräsentiert das erste Referenzsignal 131 erste optofluidische Modelldaten repräsentiert, die einer ersten Eigenschaft des biologischen Materials entsprechen, und repräsentiert das zweite Referenzsignal 132 zweite optofluidische Modelldaten repräsentiert, die einer zweiten Eigenschaft des biologischen Materials entsprechen. Die Einleseeinrichtung 140 ist signalübertragungsfähig mit der Ermittlungseinrichtung 150, mit der Anwendungseinrichtung 160 und mit der Transformationseinrichtung 170 verbunden.

Die Ermittlungseinrichtung 150 ist ausgebildet, um ein Rauschen des Messsignals 120 zu ermitteln. Ferner ist die Ermittlungseinrichtung 150 ausgebildet, um Rauschdaten 155 zu erzeugen, welche das ermittelte Rauschen des Messsignals 120 repräsentieren. Die Ermittlungseinrichtung 150 ist signalübertragungsfähig mit der Anwendungseinrichtung 160 verbunden.

Die Anwendungseinrichtung 160 ist ausgebildet, um die ermittelten Rauschdaten 155 auf das erste Referenzsignal 131 und auf das zweite Referenzsignal 132 anzuwenden, um ein angepasstes erstes Referenzsignal 161 und ein angepasstes zweites Referenzsignal 162 zu generieren. Die Anwendungseinrichtung 160 ist signalübertragungsfähig mit der Transformationseinrichtung 170 verbunden.

Die Transformationseinrichtung 170 ist ausgebildet, um das Messsignal 120, das angepasste erste Referenzsignal 161 und das angepasste zweite Referenzsignal 162 in eine Häufigkeitsverteilungsform zu transformieren, um eine Messsignalverteilung 175, eine erste Referenzverteilung 171 und eine zweite Referenzverteilung 172 zu erzeugen. Die Transformationseinrichtung 170 ist signalübertragungsfähig mit der Durchführungseinrichtung 180 verbunden.

Die Durchführungseinrichtung 180 ist ausgebildet, um unter Verwendung der Messsignalverteilung 175, der ersten Referenzverteilung 171 und der zweiten Referenzverteilung 172 eine Clusteranalyse durchzuführen. Genauer gesagt ist die Durchführungseinrichtung 180 ausgebildet, um die Clusteranalyse durchzuführen, um abhängig von einem Resultat der Clusteranalyse festzustellen, ob das biologische Material die erste Eigenschaft oder die zweite Eigenschaft aufweist. Das Resultat der Clusteranalyse repräsentiert eine Zugehörigkeit der Messsignalverteilung 175 zu der ersten Referenzverteilung 171 oder zu der zweiten Referenzverteilung 172.

Das Steuergerät 130 ist ferner ausgebildet, um ein Analysesignal 190 auszugeben oder bereitzustellen. Das Analysesignal 190 repräsentiert ein Ergebnis der Analyse. Beispielsweise umfasst das Analysesignal eine Information über die Eigenschaft des biologischen Materials.

Gemäß einem Ausführungsbeispiel ist das Steuergerät 130 auch ausgebildet, um das eingelesene Messsignal 120 zu normieren, insbesondere durch Projizieren von Absolutwerten des Messsignals 120 auf ein vordefiniertes Werteintervall. Optional ist die Normierung mittels der Einleseeinrichtung 140 oder der Ermittlungseinrichtung 150 ausführbar. Hierbei ist die Ermittlungseinrichtung 150 ausgebildet, um das Rauschen anhand des normierten Messsignals zu ermitteln.

Insbesondere die durch das Steuergerät 130 ausgeführten Vorgänge werden unter Bezugnahme auf die nachfolgenden Figuren noch weiter verdeutlicht.

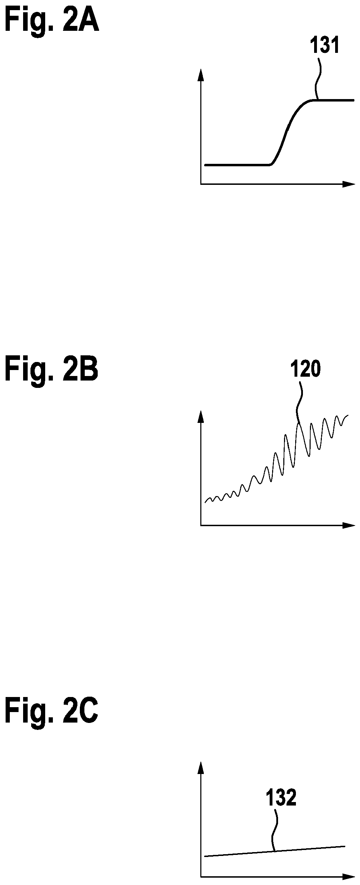

Fig. 2A, Fig. 2B und Fig. 2C zeigen schematische Signaldiagramme gemäß einem Ausführungsbeispiel. Hierbei zeigt Fig. 2A das erste Referenzsignal 131 aus Fig. 1. Das erste Referenzsignal 131 weist einen sigmoidalen Verlauf bzw. Sigma-Verlauf auf. Fig. 2B zeigt das Messsignal 120 aus Fig. 1. Das Messsignal 120 ist mit Rauschen behaftet. Fig. 2C zeigt das zweite Referenzsignal 132 aus Fig. 1. Das zweite Referenzsignal 132 weist einen linearen Verlauf auf.

Anders ausgedrückt zeigen Fig. 2A, Fig. 2B und Fig. 2C ein Entscheidungsproblem einer qPCR-Kurvenauswertung. Wurde eine qPCR-Kurve in Gestalt des Messsignals 120 bzw. in Gestalt von Rohdaten aufgezeichnet, wird diese dahingehend bewertet, ob es sich um eine Amplifikation oder keine Amplifikation handelt. Das erste Referenzsignal 131 repräsentiert die Amplifikation. Das zweite Referenzsignal 132 repräsentiert keine Amplifikation bzw. ein Ausbleiben einer Amplifikation. Das erste Referenzsignal 131 kann als eine sigmoidale Funktion mit fünf Parametern, als nicht linear und mit S-Form beschrieben werden. Das zweite Referenzsignal 132 kann als eine lineare Funktion mit zwei Parametern beschrieben werden. Das Messsignal 120 basiert üblicherweise auf n Messpunkten, wobei n einer Anzahl PCR-Zyklen der Analyse entspricht. Systembedingt weisen diese Messpunkte ein Rauschen η auf. Es besteht dann das Entscheidungsproblem, ob das Messsignal 120 mit dem Rauschen η eher mit dem ersten Referenzsignal 131 oder mit dem zweiten Referenzsignal 132 übereinstimmt bzw. Ähnlichkeit hat.

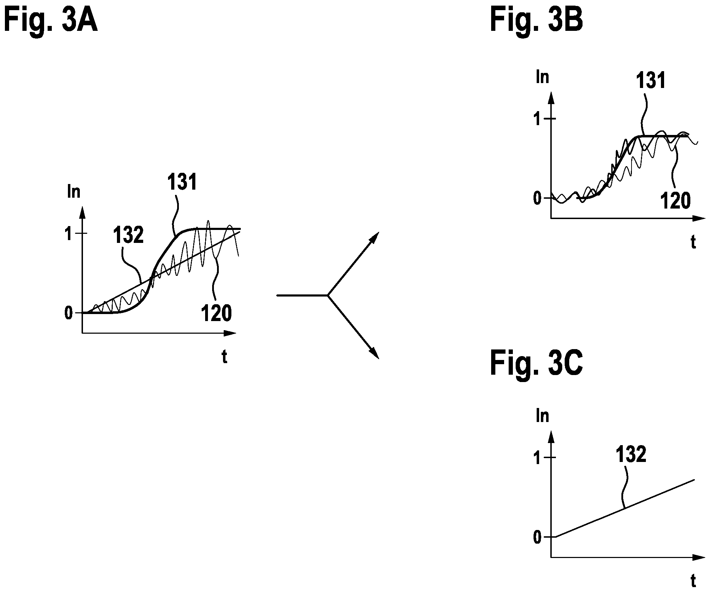

Fig. 3A, Fig. 3B und Fig. 3C zeigen schematische Signaldiagramme gemäß einem Ausführungsbeispiel. Die schematischen Signaldiagramme sind in Gestalt von Intensität-Zeit-Diagrammen bzw. Intensität-Zyklus-Diagrammen dargestellt. Hierbei ist an den Abszissenachsen jeweils die Zeit t bzw. eine Zykluszahl aufgetragen. An den Ordinatenachsen ist jeweils eine Intensität In bzw. Signalintensität In aufgetragen. Fig. 3A zeigt das Messsignal 120 aus Fig. 2B, das erste Referenzsignal 131 aus Fig. 2A und das zweite Referenzsignal 132 aus Fig. 2C in überlagerter Form. Fig. 3B zeigt das Messsignal 120 und das erste Referenzsignal 131 in überlagerter Form. Fig. 3C zeigt das zweite Referenzsignal 132. Dabei sind in Fig. 3A, Fig. 3B und Fig. 3C das Messsignal 120, das erste Referenzsignal 131 und das zweite Referenzsignal 132 bezogen auf die Intensität In normiert, beispielsweise auf ein Intervall zwischen 0 und 1.

Genauer gesagt und anders ausgedrückt zeigen Fig. 3A, Fig. 3B und Fig. 3C einen Ansatz zur Lösung des in Verbindung mit Fig. 2A, Fig. 2B und Fig. 2C erwähnten Entscheidungsproblems. Anstelle einer Ausgleichsrechnung des Messsignals 120 gegen das erste Referenzsignal 131 und das zweite Referenzsignal 132 und einer Abschätzung des Fehlers (Goodness-of-Fit oder Kogolomorow-Smirnow-Test), welcher bei großem Rauschen η, das in mikrofluidischen Vorrichtungen und Systemen durch Luftblasen verstärkt werden kann, wird hier auf eine Clusteranalyse bzw. ein Clusterverfahren (k-means-clustering) unter Verwendung des k-Means-Algorithmus zurückgegriffen, um eine Robustheit der Analyse des biologischen Materials beizubehalten. Dazu wird das Messsignal 120 normiert, d. h. Absolutwerte auf ein Intervall [0,1] projiziert, und mit zwei generischen Funktionen, welche dem ersten Referenzsignal 131 und dem zweiten Referenzsignal 132 entsprechen, verglichen. Der Vergleich ist dabei ein sogenanntes k-means-Clustering mit einem vordefinierten Distanzmaß, die beispielsweise Euler-Distanz, Manhattan-Distanz oder dergleichen, mit insgesamt zwei Clustern, k=2. Dabei bilden die generischen Funktionen (auch Dummy Functions genannt) jeweils eine Clusterkategorie bzw. ein Cluster und das Messsignal 120 fällt durch die jeweils kleinere Distanz in eines dieser Cluster. Es ist möglich, dass das Messsignal 120 ein eigenes Cluster bildet und die auf den Referenzsignalen 131 und 132 beruhenden Cluster zusammenfallen. System- und theoriebedingt würde hier eine invalide Messung vorliegen, da das Messsignal 120 eine nicht zu erwartende Form aufweist. Dieser Fall kann als Qualitätskontrolle eingesetzt werden.

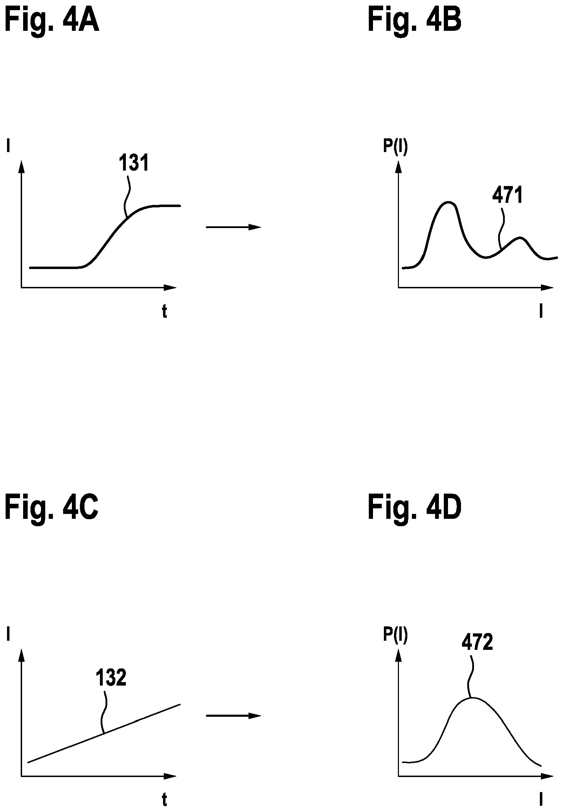

Fig. 4A, Fig. 4B, Fig. 4C und Fig. 4D zeigen schematische Signaldiagramme gemäß einem Ausführungsbeispiel. Die schematischen Signaldiagramme in Fig. 4A und Fig. 4C sind in Gestalt von Intensität-Zeit-Diagrammen bzw. Intensität-Zyklus-Diagrammen dargestellt. Hierbei ist an den Abszissenachsen jeweils die Zeit t bzw. eine Zykluszahl aufgetragen. An den Ordinatenachsen ist jeweils eine Intensität I bzw. Signalintensität I aufgetragen. Die schematischen Signaldiagramme in Fig. 4B und Fig. 4D sind in Gestalt von Intensitätsverteilung-Intensität-Diagrammen dargestellt. Hierbei ist an den Abszissenachsen jeweils die Intensität I bzw. Signalintensität I aufgetragen. An den Ordinatenachsen ist jeweils eine Intensitätsverteilung P(I) aufgetragen. Fig. 4A zeigt das erste Referenzsignal 131 aus einer der vorstehend genannten Figuren. Fig. 4B zeigt eine erste Referenzverteilung 471, die durch Transformieren des ersten Referenzsignals 131 in eine Verteilungsform erhalten wird. Fig. 4C zeigt das zweite Referenzsignal 132 aus einer der vorstehend genannten Figuren. Fig. 4D zeigt eine zweite Referenzverteilung 472, die durch Transformieren des zweiten Referenzsignals 132 in eine Verteilungsform erhalten wird.

Anders ausgedrückt zeigen Fig. 4A, Fig. 4B, Fig. 4C und Fig. 4D einen ersten Schritt zum robusten Clusterentscheid. Anstatt die qPCR bzw. das daraus hervorgehende Messsignal in Form einer Zyklus-Intensität-Abhängigkeit zu beurteilen, wird ein Histogramm von Intensitätswerten erstellt. Dazu wird eine Kerneldichte-Abschätzung über das Messsignal bzw. die PCR-Kurve durchgeführt. Da eine qPCR-Kurve, amplifizierend oder nicht-amplifizierend, monoton-steigend (mit Rauschen im Trend monoton steigend) ist, korrespondieren tiefe Intensitäten mit frühen Zyklen und hohe Intensitäten mit späten Werten. Wird nun das erste Referenzsignal 131 transformiert, entsteht eine bimodale Verteilung als erste Referenzverteilung 471. Dies ist durch die zwei Plateaus der sigmoidalen Kurve des ersten Referenzsignals 131 erklärbar. Die lineare Funktion des zweiten Referenzsignals 132 hingegen weist eine unimodale Verteilung der Intensitäten als zweite Referenzverteilung 472 auf. Durch eine solche Transformation reduziert sich das Entscheidungsproblem auf das Feststellen einer unimodalen oder bimodalen Verteilung in einem entsprechend in die Verteilungsform transformierten Messsignal.

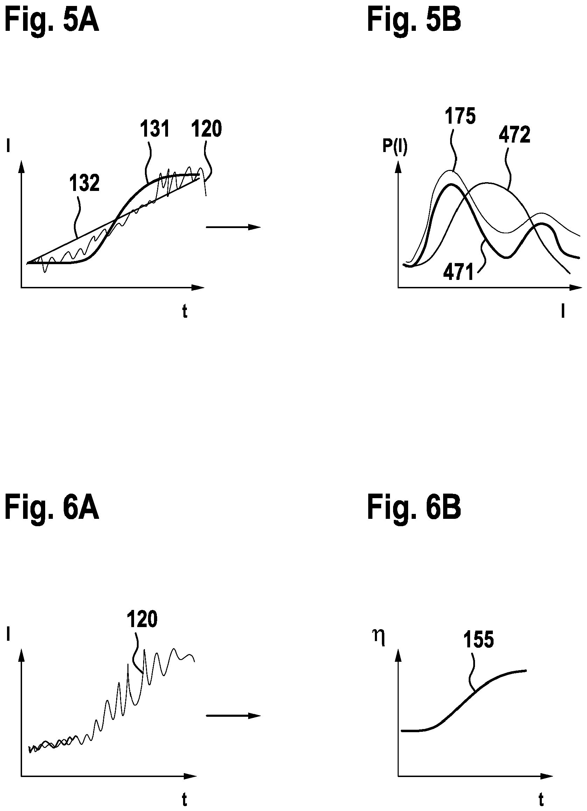

Fig. 5A und Fig. 5B zeigen schematische Signaldiagramme gemäß einem Ausführungsbeispiel. Das schematische Signaldiagramm in Fig. 5A ist in Gestalt eines Intensität-Zeit-Diagramms bzw. Intensität-Zyklus-Diagramms dargestellt. Hierbei ist an der Abszissenachse die Zeit t bzw. eine Zykluszahl aufgetragen. An der Ordinatenachse ist eine Intensität I bzw. Signalintensität I aufgetragen. Fig. 5A zeigt das Messsignal 120, das erste Referenzsignal 131 und das zweite Referenzsignal 132 aus einer der vorstehend genannten Figuren in überlagerter Form. Das schematische Signaldiagramm in Fig. 5B ist in Gestalt eines Intensitätsverteilung-Intensität-Diagramms dargestellt. Hierbei ist an der Abszissenachse die Intensität I bzw. Signalintensität I aufgetragen. An der Ordinatenachse ist eine Intensitätsverteilung P(I) aufgetragen. Fig. 5B zeigt die Messsignalverteilung 175 aus Fig. 1, die erste Referenzverteilung 471 aus Fig. 4B und die zweite Referenzverteilung 472 aus Fig. 4D, die jeweils durch Transformieren in eine Verteilungsform erhalten werden.

Anders ausgedrückt werden das Messsignal 120, das erste Referenzsignal 131 und das zweite Referenzsignal 132 in Intensitätsverteilungen transformiert. Diese Verteilungen werden nun einer Clusteranalyse mit k=2 unterzogen. Die bimodale und die unimodale Verteilung der generischen Funktionen bzw. Referenzverteilungen 471 und 472 bilden robust zwei Cluster, wobei die Messsignalverteilung 175 bzw. Rohdatenverteilung in eine der beiden Cluster bzw. Kategorien fällt, abhängig von deren Modalität.

Fig. 6A und Fig. 6B zeigen schematische Signaldiagramme gemäß einem Ausführungsbeispiel. Das schematische Signaldiagramm in Fig. 6A ist in Gestalt eines Intensität-Zeit-Diagramms bzw. Intensität-Zyklus-Diagramms dargestellt. Hierbei ist an der Abszissenachse die Zeit t bzw. eine Zykluszahl aufgetragen. An der Ordinatenachse ist eine Intensität I bzw. Signalintensität I aufgetragen. Fig. 6A zeigt das Messsignal 120 aus einer der vorstehend genannten Figuren. Das schematische Signaldiagramm in Fig. 6B ist in Gestalt eines Rausch-Zeit-Diagramms dargestellt. Hierbei ist an der Abszissenachse die Zeit t bzw. eine Zykluszahl aufgetragen. An der Ordinatenachse ist ein Rauschen η aufgetragen. Fig. 6B zeigt die Rauschdaten 155 aus Fig. 1 bzw. das ermittelte Rauschen des Messsignals.

Anders ausgedrückt zeigen Fig. 6A und Fig. 6B einen weiteren Schritt zur Erlangung robuster Entscheidungen. Wie in Fig. 4A, Fig. 4B, Fig. 4C und Fig. 4D sowie Fig. 5A und Fig. 5B gezeigt, kann ein Messsignal 120 bzw. eine qPCR-Kurve in der Verteilungsform bzw. Intensitätsverteilung mit wenig Aufwand als amplifizierend oder nicht amplifizierend charakterisiert werden. Ist nun aber das Rauschen η hoch, so wird die Verteilung der Intensitäten entsprechend gestreckt und gestaucht. Die Momente der Verteilungskurven, beispielsweise Erwartungswerte/Spitzen, Breite der Spitzen/Varianzen und Symmetrie der Kurve/Schiefe (Skewness) weichen von Idealkurven ab und werden weniger prominent, d. h. anspruchsvoller zu charakterisieren. Das vorhandene Rauschen η ist allerdings eine messbare Größe, welche sich aus den Rohdaten des Messsignals 120 ermitteln lässt. Da Luftblasen in mikrofluidischen Vorrichtungen oder Systemen einen großen Störbeitrag leisten, ist das Rauschen η in Abhängigkeit von der Zyklenzahl bzw. Zeit t nicht konstant, sondern nimmt mit größerer Zyklenzahl bzw. fortschreitender Zeit t zu. Dies ist darauf zurückzuführen, dass Anzahl und Größe von Luftblasen über die Reaktionszeit der Analyse grösser werden. Mit einem gleitenden Fensteransatz (sliding window approach) wird das Rauschen η in Abhängigkeit von der Zyklenzahl bzw. der Zeit t mit einem geeigneten Rauschmaß gemessen, z. B. lokale Standardabweichung, lokales Signal-zu-Rausch-Verhältnis oder dergleichen. Daraus werden die Rauschdaten 155 erhalten, welche den funktionalen Zusammenhang von Rauschen η und Zyklenzahl bzw. Zeit t für die vorliegende Analyse zeigen.

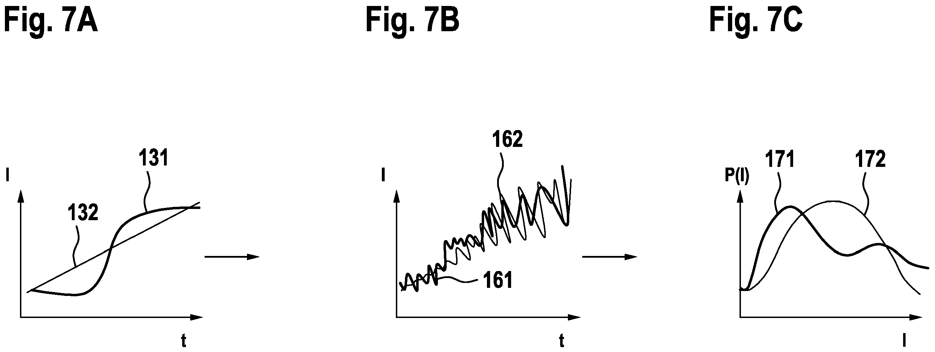

Fig. 7A, Fig. 7B und Fig. 7C zeigen schematische Signaldiagramme gemäß einem Ausführungsbeispiel. Die schematischen Signaldiagramme in Fig. 7A und Fig. 7B sind in Gestalt von Intensität-Zeit-Diagrammen bzw. Intensität-Zyklus-Diagrammen dargestellt. Hierbei ist an den Abszissenachsen jeweils die Zeit t bzw. eine Zykluszahl aufgetragen. An den Ordinatenachsen ist jeweils eine Intensität I bzw. Signalintensität I aufgetragen. Fig. 7A zeigt das erste Referenzsignal 131 und das zweite Referenzsignal 132 aus einer der vorstehend beschriebenen Figuren. Fig. 7B zeigt das angepasste erste Referenzsignal 161 und das angepasste zweite Referenzsignal 162 aus Fig. 1 Das schematische Signaldiagramm in Fig. 7C ist in Gestalt eines Intensitätsverteilung-Intensität-Diagramms dargestellt. Hierbei ist an der Abszissenachse die Intensität I bzw. Signalintensität I aufgetragen. An der Ordinatenachse ist eine Intensitätsverteilung P(I) aufgetragen. Fig. 7C zeigt die erste Referenzverteilung 171 und die zweite Referenzverteilung 172 aus Fig. 1, die durch Transformieren der jeweiligen angepassten Referenzsignale 161 und 162 in eine Verteilungsform erhalten werden.

Anders ausgedrückt zeigen Fig. 7A, Fig. 7B und Fig. 7C, wie der Rauschgraph bzw. die Rauschdaten 155 aus Fig. 6B genutzt werden, um die Referenzsignale 131 und 132 mit denselben Rauschverhältnissen zu aktualisieren, denen das Messsignal unterliegt. Die Referenzsignale 131 und 132 repräsentieren Modelldaten mit idealen Werten, wobei zu jedem idealen Wert für jeden Zyklus der Analyse eine Zufallszahl bzw. Pseudozufallszahl hinzuaddiert wird. Diese Zufallszahl bzw. Pseudozufallszahl wird aus dem Rauschintervall des in Fig. 6A gemessenen Signals bzw. aus den Rauschdaten aus Fig. 6B generiert. Dadurch entstehen zwei angepasste Referenzsignale 161 und 162, welche dasselbe Rauschen wie das Messsignal aufweisen. Werden die angepassten Referenzsignale 161 und 162 in die Verteilungsform bzw. Intensitätsverteilung transformiert, werden die idealen Verteilungen dem gemessenen Rauschen entsprechend verformt, insbesondere gestaucht. Für die Clusteranalyse sind somit das Messsignal und die passende Referenzverteilung 171 oder 172 näher und ist der Clusterentscheid robuster.

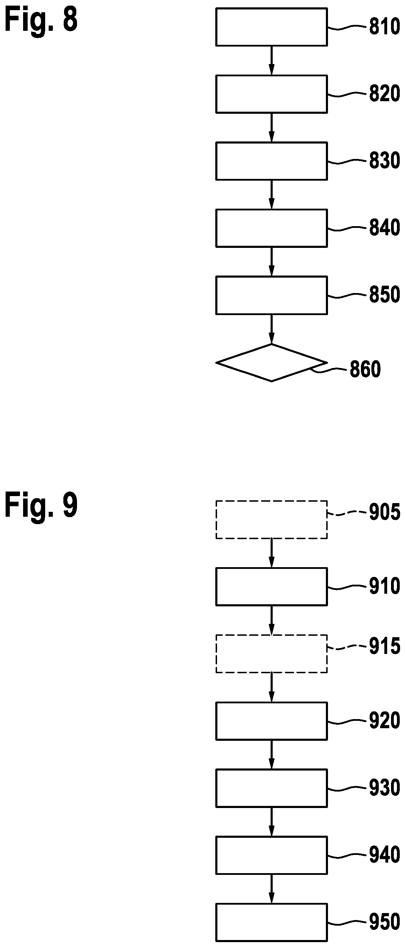

Fig. 8 zeigt ein Ablaufdiagramm eines Auswerteprozesses 800 gemäß einem Ausführungsbeispiel. Dabei ist der Auswerteprozess 800 in Verbindung mit der Vorrichtung aus Fig. 1 oder einer ähnlichen Vorrichtung ausführbar. Ein erster Block 810 des Auswerteprozesses 800 repräsentiert das Messsignal. Ein nachfolgender zweiter Block 820 repräsentiert ein Normieren des Messsignals und ein Messen eines zyklusabhängigen Rauschens η(t). Ein nachfolgender dritter Block 830 repräsentiert ein Aktualisieren des ersten Referenzsignals und des zweiten Referenzsignals mit dem Rauschen η(t). Ein wiederum nachfolgender vierter Block 840 repräsentiert ein Transformieren des Messsignals sowie der aktualisierten Referenzsignale in eine Verteilungsform. Ein nachfolgender fünfter Block 850 repräsentiert eine Clusteranalyse unter Verwendung des transformierten Messsignals sowie der transformierten aktualisierten Referenzsignal mittels eines k-Means-Algorithmus mit k=2. Ein abschließender sechster Block 860 repräsentiert eine Entscheidung gemäß der Clusteranalyse dahingehend, ob das Messsignal auf eine Amplifikation hindeutet oder nicht.

Der Auswerteprozess 800 kann auch als ein robuster Entscheidungsprozess bezeichnet werden. Der Auswerteprozess 800 ist insbesondere geeignet zur Auswertung von qPCR mit hohem Rauschen. Als Eingangsdaten des Auswerteprozesses 800 dient eine qPCR-Kurve in Gestalt des Messsignals. Das Messsignal wird in einem ersten Schritt normiert und das zyklenabhängige Rauschen wird gemessen, wie es in dem zweiten Block 820 gezeigt ist. Als nächstes werden zwei Referenzsignale bzw. generische Kurven mit dem gemessenen Rauschen erzeugt bzw. aktualisiert, wie es in dem dritten Block 830 gezeigt ist. Das erste Referenzsignal weist einen qPCR-typischen sigmodialen Verlauf auf und das zweite Referenzsignal weist einen linearen Verlauf auf. Das Messsignal, das erste Referenzsignal und das zweite Referenzsignal, welche auf die zuvor beschriebene Weise verarbeitet wurden, werden dann in eine Intensitätsverteilung umgewandelt, wie es in dem vierten Block 840 gezeigt ist. Nachfolgend werden die umgewandelten Signale mittels Clusteranalyse in zwei Cluster eingeteilt, wie es in dem fünften Block 850 gezeigt ist. Das Messsignal wird dann weiterbehandelt wie das Referenzsignal, dem das Messsignal bei der Clusteranalyse zugeordnet wurde. Der Auswerteprozess 800 kann direkt in eine Auswertesoftware einer mikrofluidischen Vorrichtung, wie der Vorrichtung aus Fig. 1, integriert sein oder werden.

Fig. 9 zeigt ein Ablaufdiagramm eines Verfahrens 900 bzw. Analyseverfahrens 900 gemäß einem Ausführungsbeispiel. Das Verfahren 900 ist ausführbar, um eine Analyse von biologischem Material durchzuführen. Dabei ist das Verfahren 900 in Verbindung mit der Vorrichtung aus Fig. 1 oder einer ähnlichen Vorrichtung ausführbar.

In einem Schritt 910 des Einlesens werden bei dem Verfahren 900 zur Analyse ein Messsignal, ein erstes Referenzsignal und ein zweites Referenzsignal eingelesen. Das Messsignal repräsentiert erfasste optofluidische Daten des biologischen Materials. Das erste Referenzsignal repräsentiert erste optofluidische Modelldaten, die einer ersten Eigenschaft des biologischen Materials entsprechen. Das zweite Referenzsignal repräsentiert zweite optofluidische Modelldaten repräsentiert, die einer zweiten Eigenschaft des biologischen Materials entsprechen.

Nachfolgend wird bei dem Verfahren 900 zur Analyse in einem Schritt 920 des Ermittelns ein Rauschen des eingelesenen Messsignals ermittelt, um Rauschdaten zu erzeugen. Wiederum nachfolgend werden in einem Schritt 930 des Anwendens die Rauschdaten auf das erste Referenzsignal und auf das zweite Referenzsignal angewendet, um ein angepasstes erstes Referenzsignal und ein angepasstes zweites Referenzsignal zu generieren. Nachfolgend werden in einem Schritt 940 des Transformierens das Messsignal, das angepasste erste Referenzsignal und das angepasste zweite Referenzsignal in eine Häufigkeitsverteilungsform transformiert, um eine Messsignalverteilung, eine erste Referenzverteilung und eine zweite Referenzverteilung zu erzeugen.

Nachfolgend wird bei dem Verfahren 900 zur Analyse in einem Schritt 950 des Durchführens eine Clusteranalyse unter Verwendung bzw. an der Messsignalverteilung, der ersten Referenzverteilung und der zweiten Referenzverteilung durchgeführt, um abhängig von einem Resultat der Clusteranalyse festzustellen, ob das biologische Material die erste Eigenschaft oder die zweite Eigenschaft aufweist. Auch wenn es in der Darstellung von Fig. 9 nicht gezeigt ist, kann das Verfahren 900 zur Analyse auch einen Schritt des Ausgebens oder Bereitstellens eines Analysesignals aufweisen, das ein Ergebnis der Analyse repräsentiert.

Gemäß einem Ausführungsbeispiel weist das Verfahren 900 zur Analyse einen Schritt 905 des Erfassens der optofluidischen Daten des biologischen Materials auf, um das Messsignal bereitzustellen. Dabei ist der Schritt 905 des Erfassens vor dem Schritt 910 des Einlesens ausführbar.

Optional weist das Verfahren 900 zur Analyse einen Schritt 915 des Normierens des eingelesenen Messsignals durch Projizieren von Absolutwerten auf ein vordefiniertes Werteintervall auf. Der Schritt 915 des Normierens ist dabei vor dem Schritt 920 des Ermittelns ausführbar. Im Schritt 920 des Ermittelns wird hierbei das Rauschen des normierten Messsignals ermittelt.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (900) zur Analyse von biologischem Material, wobei das Verfahren (900) folgende Schritte aufweist:
Einlesen (910) eines Messsignals (120), eines ersten Referenzsignals (131) und eines zweiten Referenzsignals (132), wobei das Messsignal (120) erfasste optofluidische Daten des biologischen Materials repräsentiert, wobei das erste Referenzsignal (131) mit sigmoidalem Verlauf erste optofluidische Modelldaten repräsentiert, die einer ersten Eigenschaft des biologischen Materials entsprechen, wobei das zweite Referenzsignal (132) mit linearem Verlauf zweite optofluidische Modelldaten repräsentiert, die einer zweiten Eigenschaft des biologischen Materials entsprechen;
Ermitteln (920) eines Rauschens (η) des Messsignals (120), um Rauschdaten (155) zu erzeugen;
Anwenden (930) der Rauschdaten (155) auf das erste Referenzsignal (131) und auf das zweite Referenzsignal (132), um ein angepasstes erstes Referenzsignal (161) und ein angepasstes zweites Referenzsignal (162) zu generieren;
Transformieren (940) des Messsignals (120), des angepassten ersten Referenzsignals (161) und des angepassten zweiten Referenzsignals (162) in eine Häufigkeitsverteilungsform, um eine Messsignalverteilung (175), eine erste bimodale Referenzverteilung (171) und eine zweite unimodale Referenzverteilung (172) zu erzeugen; und
Durchführen (950) einer Clusteranalyse unter Verwendung der Messsignalverteilung (175), der ersten Referenzverteilung (171) und der zweiten Referenzverteilung (172), um abhängig von einem Resultat der Clusteranalyse festzustellen, ob das biologische Material die erste Eigenschaft oder die zweite Eigenschaft aufweist.

2. Verfahren (900) gemäß Anspruch 1, mit einem Schritt (905) des Erfassens der optofluidischen Daten des biologischen Materials, um das Messsignal (120) bereitzustellen.

3. Verfahren (900) gemäß einem der vorangegangenen Ansprüche, bei dem im Schritt (910) des Einlesens ein Messsignal (120) eingelesen wird, das mittels einer quantitativen und/oder qualitativen Polymerase-Kettenreaktion erfasste optofluidische Daten des biologischen Materials repräsentiert, wobei im Schritt (910) des Einlesens ein erstes Referenzsignal (131), das einen sigmoidalen Verlauf aufweist, und ein zweites Referenzsignal (132), das einen linearen Verlauf aufweist, eingelesen werden, wobei die erste Eigenschaft des biologischen Materials zu einer Amplifikation zumindest eines Zielmoleküls des biologischen Materials durch die Analyse führt, wobei die zweite Eigenschaft des biologischen Materials zu einem Ausbleiben einer Amplifikation des zumindest einen Zielmoleküls durch die Analyse führt, wobei im Schritt (940) des Transformierens das angepasste erste Referenzsignal (161) und das angepasste zweite Referenzsignal (162) derart transformiert werden, dass die erste Referenzverteilung (171) eine bimodale Verteilung ist und die zweite Referenzverteilung (172) eine unimodale Verteilung ist.

4. Verfahren (900) gemäß einem der vorangegangenen Ansprüche, bei dem im Schritt (950) des Durchführens die Clusteranalyse mittels eines k-Means-Algorithmus mit einem vordefinierten Distanzmaß durchgeführt wird, wobei die erste Referenzverteilung (171) ein erstes Cluster repräsentiert, wobei die zweite Referenzverteilung (172) ein zweites Cluster repräsentiert, wobei das Resultat der Clusteranalyse angibt, ob unter Berücksichtigung des Distanzmaßes die Messsignalverteilung (175) in das erste Cluster oder in das zweite Cluster fällt.

5. Verfahren (900) gemäß einem der vorangegangenen Ansprüche, bei dem im Schritt (920) des Ermittelns das Rauschen (η) des Messsignals (120) über einen Verlauf der Analyse hinweg und/oder mittels eines Schiebefensterprozesses und/oder unter Verwendung eines Rauschmaßes ermittelt wird, um als die Rauschdaten (155) einen funktionalen Zusammenhang zwischen dem Rauschen (η) und dem Verlauf der Analyse zu erzeugen.

6. Verfahren (900) gemäß einem der vorangegangenen Ansprüche, bei dem im Schritt (930) des Anwendens zu den Modelldaten der Referenzsignale (131, 132) von den Rauschdaten (155) abhängige Zufallszahlen oder Pseudozufallszahlen addiert werden.

7. Verfahren (900) gemäß einem der vorangegangenen Ansprüche, mit einem Schritt (915) des Normierens des eingelesenen Messsignals (120) durch Projizieren von Absolutwerten auf ein vordefiniertes Werteintervall, wobei im Schritt (920) des Ermittelns das (η) Rauschen des normierten Messsignals (120) ermittelt wird.

8. Vorrichtung (100), die eingerichtet ist, um die Schritte des Verfahrens (900) gemäß einem der vorangegangenen Ansprüche in entsprechenden Einheiten (110, 115, 130, 140, 150, 160, 170, 180) auszuführen und/oder anzusteuern.

9. Computerprogramm, das dazu eingerichtet ist, die Schritte des Verfahrens (900) gemäß einem der vorangegangenen Ansprüche auszuführen und/oder anzusteuern.

10. Maschinenlesbares Speichermedium, auf dem das Computerprogramm nach Anspruch 9 gespeichert ist.

## Claims

1. Method (900) for analyzing biological material, wherein the method (900) includes the following steps:
reading (910) a measurement signal (120), a first reference signal (131), and a second reference signal (132), wherein the measurement signal (120) represents acquired optofluidic data of the biological material, wherein the first reference signal (131) having a sigmoid curve represents first optofluidic model data which correspond to a first property of the biological material, wherein the second reference signal (132) having a linear curve represents second optofluidic model data which correspond to a second property of the biological material;
ascertaining (920) a noise (η) of the measurement signal (120), to generate noise data (155);
applying (930) the noise data (155) to the first reference signal (131) and to the second reference signal (132), to generate an adapted first reference signal (161) and an adapted second reference signal (162) ;
transforming (940) the measurement signal (120), the adapted first reference signal (161), and the adapted second reference signal (162) into a frequency distribution form to generate a measurement signal distribution (175), a first bimodal reference distribution (171), and a second unimodal reference distribution (172); and
performing (950) a cluster analysis using the measurement signal distribution (175), the first reference distribution (171), and the second reference distribution (172) to establish as a function of a result of the cluster analysis whether the biological material has the first property or the second property.

2. Method (900) according to Claim 1, having a step (905) of acquiring the optofluidic data of the biological material to provide the measurement signal (120).

3. Method (900) according to either of the preceding claims, in which, in the step (910) of reading, a measurement signal (120) is read which represents optofluidic data of the biological material acquired by means of a quantitative and/or qualitative polymerase chain reaction, wherein in the step (910) of reading, a first reference signal (131), which has a sigmoid curve, and a second reference signal (132), which has a linear curve, are read, wherein the first property of the biological material results in an amplification of at least one target molecule of the biological material by the analysis, wherein the second property of the biological material results in an absence of an amplification of the at least one target molecule by the analysis, wherein in the step (940) of transforming, the adapted first reference signal (161) and the adapted second reference signal (162) are transformed in such a way that the first reference distribution (171) is a bimodal distribution and the second reference distribution (172) is a unimodal distribution.

4. Method (900) according to any one of the preceding claims, in which, in the step (950) of performing, the cluster analysis is performed by means of a k-means algorithm having a predefined distance measure, wherein the first reference distribution (171) represents a first cluster, wherein the second reference distribution (172) represents a second cluster, wherein the result of the cluster analysis specifies whether, in consideration of the distance measure, the measurement signal distribution (175) falls into the first cluster or into the second cluster.

5. Method (900) according to any one of the preceding claims, in which, in the step (920) of ascertaining, the noise (η) of the measurement signal (120) is ascertained over a curve of the analysis and/or by means of a sliding window process and/or using a noise measure to generate as the noise data (155) a functional relationship between the noise (η) and the curve of the analysis.

6. Method (900) according to any one of the preceding claims, in which, in the step (930) of applying, random numbers or pseudorandom numbers dependent on the noise data (155) are added to the model data of the reference signals (131, 132).

7. Method (900) according to any one of the preceding claims, having a step (915) of scaling the read measurement signal (120) by projecting absolute values on a predefined value interval, wherein, in the step (920) of ascertaining, the noise (η) of the scaled measurement signal (120) is ascertained.

8. Device (100), which is configured to execute and/or activate the steps of the method (900) according to any one of the preceding claims in corresponding units (110, 115, 130, 140, 150, 160, 170, 180).

9. Computer program, which is configured to execute and/or activate the steps of the method (900) according to any one of the preceding claims.

10. Machine-readable storage medium, on which the computer program according to Claim 9 is stored.

## Revendications

1. Procédé (900) d'analyse de matériel biologique, le procédé (900) comprenant les étapes suivantes :
lecture (910) d'un signal de mesure (120), d'un premier signal de référence (131) et d'un deuxième signal de référence (132), le signal de mesure (120) représentant des données optofluidiques acquises du matériel biologique, le premier signal de référence (131), avec un tracé sigmoïdal, représentant des premières données modèles optofluidiques qui correspondent à une première propriété du matériel biologique, le deuxième signal de référence (132), avec un tracé linéaire, représentant des deuxièmes données modèles optofluidiques qui correspondent à une deuxième propriété du matériel biologique ;
détermination (920) d'un bruit (η) du signal de mesure (120) afin de générer des données de bruit (155) ;
application (930) des données de bruit (155) sur le premier signal de référence (131) et sur le deuxième signal de référence (132) afin de générer un premier signal de référence adapté (161) et un deuxième signal de référence adapté (162) ;
transformation (940) du signal de mesure (120), du premier signal de référence adapté (161) et du deuxième signal de référence adapté (162) en une forme de distribution de la fréquence afin de générer une distribution du signal de mesure (175), une première distribution de référence (171) bimodale et une deuxième distribution de référence (172) unimodale ; et
réalisation (950) d'une analyse par groupe en utilisant la distribution du signal de mesure (175), la première distribution de référence (171) et la deuxième distribution de référence (172) afin de constater, à partir d'un résultat de l'analyse par groupe, si le matériel biologique présente la première propriété ou la deuxième propriété.

2. Procédé (900) selon la revendication 1, comprenant une étape (905) d'acquisition des données optofluidiques du matériel biologique afin de fournir le signal de mesure (120).

3. Procédé (900) selon l'une des revendications précédentes, avec lequel, à l'étape (910) de lecture, un signal de mesure (120) est lu, lequel représente des données optofluidiques du matériel biologique acquises au moyen d'une réaction en chaîne par polymérase quantitative et/ou qualitative, un premier signal de référence (131), qui présente un tracé sigmoïde, et un deuxième signal de référence (132), qui présente un tracé linéaire, étant lus à l'étape (910) de lecture, la première propriété du matériel biologique entraînant une amplification d'au moins une molécule cible du matériel biologique par l'analyse, la deuxième propriété du matériel biologique entraînant une absence de l'amplification de l'au moins une molécule cible du matériel biologique par l'analyse, le premier signal de référence adapté (161) et le deuxième signal de référence adapté (162) étant transformés à l'étape (940) de transformation de telle sorte que la première distribution de référence (171) soit une distribution bimodale et la deuxième distribution de référence (172) soit une distribution unimodale.

4. Procédé (900) selon l'une des revendications précédentes, avec lequel, à l'étape (950) de réalisation, l'analyse par groupe est réalisée au moyen d'un algorithme des k moyennes avec une cote de distance prédéfinie, la première distribution de référence (171) représentant un premier groupe, la deuxième distribution de référence (172) représentant un deuxième groupe, le résultat de l'analyse par groupe indiquant si, en prenant en considération la cote de distance, la distribution du signal de mesure (175) se retrouve dans le premier groupe ou dans le deuxième groupe.

5. Procédé (900) selon l'une des revendications précédentes, avec lequel, à l'étape (920) de détermination, le bruit (η) du signal de mesure (120) est déterminé sur un déroulement de l'analyse et/ou au moyen d'un processus de fenêtres coulissantes et/ou en utilisant un facteur de bruit afin de générer, en tant que données de bruit (155) une relation fonctionnelle entre le bruit (η) et le déroulement de l'analyse.

6. Procédé (900) selon l'une des revendications précédentes, avec lequel, à l'étape (930) de l'application, des nombres aléatoires ou des nombres pseudoaléatoires dépendants des données de bruit (155) sont ajoutés aux données modèles des signaux de référence (131, 132).

7. Procédé (900) selon l'une des revendications précédentes, comprenant une étape (915) de normalisation du signal de mesure (120) lu, par projection de valeurs absolues sur un intervalle de valeurs prédéfini, le bruit (η) du signal de mesure (120) étant déterminé à l'étape (920) de détermination.

8. Dispositif (100), qui est conçu pour exécuter et/ou commander les étapes du procédé (900) selon l'une des revendications précédentes dans des unités (110, 115, 130, 140, 150, 160, 170, 180) correspondantes.

9. Programme informatique, qui est conçu pour exécuter et/ou commander les étapes du procédé (900) selon l'une des revendications précédentes.

10. Support d'enregistrement lisible par machine sur lequel est enregistré le programme informatique selon la revendication 9.
